# EUROPEAN PATENT APPLICATION

(11) **EP 2 096 471 A1**
(43) Date of publication of application: **02.09.2009**
(21) Application number: 06841758.3
(22) Date of filing: 22.12.2006
(51) Int. Cl.: G02B 5/23, B60J 1/00, A61F 9/00

(54) **COMPONENT FOR PREVENTION PURPOSES IN HEALTHY EYES AND FOR TREATMENT AND PROPHYLAXIS IN PSEUDO-APHACIC EYES AND/OR EYES AFFECTED BY NEURODEGENERATIVE PROCESSES**

(30) Priority: 04.12.2006 ES 200603104
(71) Applicant: Universidad Complutense De Madrid, 28040 Madrid (ES)
(72) Inventor: SÁNCHEZ RAMOS, Celia, 28040 Madrid (ES)
(74) Representative: Temino Ceniceros, Ignacio
(86) International application number: PCT/ES2006/000706
(87) International publication number: WO 2008/068353

(57) **Abstract**

The subject of the invention is a transparent, filtering component for prevention purposes in healthy eyes and for treatment and prophylaxis in pseudo-aphacic eyes and/or eyes suffering from retinal and macular degeneration, **characterized in that** it is the result of applying a yellow-pigmented filter to any of the transparent or translucent surfaces of a vehicle in which the subject might be located, in order to protect same from the short wavelengths of the visible spectrum (from 500 to 380 nm). By way of example, this component may be applied to the windows of motor vehicles, buses, trains, aircraft, etc. This invention obviates the problems and risks inherent in existing techniques for providing this type of protection to healthy eyes and eyes that have undergone operations to remove cataracts and for enhancing protection for eyes affected by neurodegenerative processes, achieving this by means of the simple application of a filter to any of the transparent or translucent surfaces of any vehicle in which the subject might be located. The invention involves the combination of the transparent or translucent surface(s) of any vehicle and a yellow-pigmented filter that absorbs short wavelengths between 500 and 380 nm.

## Description

### Objective of the invention

The invention is intended for the ophthalmology sector of the market, within the area of optical applications of a therapeutic and/or prophylactic nature.

The object of this invention is a transparent filtering vehicle component for the protection of healthy eyes, pseudophakic eyes (eyes that have undergone cataract surgery) and/or eyes with macular and retinal degeneration, from short wavelengths of light. It is produced by applying a filter comprising a yellow pigment to the transparent or translucent surface/s of vehicles to protect eyes from the short wavelengths of the visible spectrum (500 to 380 nm). As an example, this component could be applied to the windscreens of cars, buses, trains, planes, and other vehicles.

### Background of the invention

Visual perception is the result of the response to visible radiation in the wavelength range 380-760 nm. In the environment, solar radiation is the main risk factor for vision. The sun emits UV rays and IR radiation, which are mainly absorbed by the atmosphere. When the solar radiation transmitted through the atmosphere reaches the Earth's surface, it consists of UV-B rays (230-300 nm), UV or UV-A rays (300-380 nm), visible light rays (380-760 nm) and IR rays (760-1400 nm). Healthy human eyes freely transmit IR rays and those of most of the visible spectrum to the retina, but the cornea and crystalline lens prevent the most reactive wavelengths of the visible spectrum (UV-B rays and the blue portion of the spectrum) from reaching the retina.

The human crystalline lens changes its transmission properties as it ages by intensifying its yellow color thus increasing its capacity to filter out UV and blue light rays. Hence, in persons older than 65 years, ultraviolet light (<400 nm) is not transmitted and the transmission of blue light (400-500 nm) is markedly reduced.

The retina is capable of protecting itself from short wavelengths of light in two ways:
through its uneven distribution of photoreceptors, such that there are no photoreceptors sensitive to blue light in the macular depression; and through the actions of yellow pigments in this zone, which also exert a protective effect.

These natural protection systems the human eye has against the shorter wavelengths of light-the crystalline lens and structures of the retina-can be seriously affected by certain diseases and/or surgical procedures:
- Cataracts, whose surgical treatment involves the removal of the crystalline lens;
- Additionally, it is common to find a pathological ageing process that causes degradation of the retinal structures producing age-related macular degeneration (AMD).

Both cataracts and AMD can coexist in persons older than 65 years. In this population of elderly subjects, cataracts are the main cause of vision loss and AMD is the main cause of blindness. In addition, an increase in both these diseases can be expected due, among other factors, to increased life expectancy. This translates into a great interest in these diseases and their treatment options in the research field and optics industry.

Several epidemiological studies have evaluated the relationship between cataract surgery and AMD. Thus, Klein (Klein R, Klein B E, Wong T Y, Tomany S C, Cruickshanks K J. The association of cataract and cataract surgery with the long-term incidence of age-related maculopathy. Arch Opthalmol 120:1551-1558.2002) and Freeman (Freeman E, Munoz B, West S K, Tielsch J M, Schein O D. Is there an association between cataract surgery and age-related macular degeneration? Am J Opthalmol 135 (6): 849-856. 2003) claim there is a higher risk of developing symptoms of AMD in persons who have undergone cataract surgery. However, in earlier investigations by Wang (Wang J J, Mitchell P, Gumming R G, Lim R. Cataract and age-related maculopathy: the Blue Mountains Eye Study. Ophthalmic Epidemiol 6: 317-326.1999) and McCarty (McCarty C A, Mukesh B N, Fu C L, Mitchell P, Wang J J, Taylor H R. Risks factors for age-related maculopathy: the Visual Impairment Project. Arch Opthalmol 119:1455-1462. 2001) this hypothesis was rejected, possibly because of the less developed technology used for their diagnostic measurements. Techniques such as optical coherence tomography that allow the accurate, rapid, and non-invasive follow up of retinal neurodegeneration processes have only recently been introduced.

These techniques are essential for monitoring the determining effect of the natural pigments that absorb harmful radiations.

Several techniques have also been developed to protect eyes subjected to cataract surgery from short wavelengths of light:
- There are several types of filter containing a yellow pigment on the market, yet there is no optimal procedure and/or device to apply these filters to the human eye as a preventive and/or therapeutic measure to replace and/or improve the eye's natural protection.
- Since the mid-1990s, eyes undergoing cataract extraction have been implanted with intraocular lenses containing a yellow pigment to act as a filter. This option requires surgical intervention with all its associated risks and difficulties. There is also a large population of subjects who have been implanted with a transparent lens to replace the natural lens during cataract surgery who are therefore devoid of the necessary protection. In these patients, the artificial crystalline lens, lacking a yellow pigment, needs to be complemented with a system to support the yellow pigment such as the vehicle component that is the object of the present invention.

Several patents related to the state of this technique have been developed (for healthy, pseudoaphakic and/or neurodegenerating eyes) although they differ considerably from the object of the present invention:
- Solar visors for a vehicle windscreen (Korean patent document no. 9 205 420 B) comprised of polarized filters, among other components.
- Windscreen photodetector (Japanese patent document no. 5 912 6935) to improve visibility in a vehicle.
- Motorcar windscreen (Japanese patent document no. 5 804 9514) that prevents direct sunlight entering the eyes without narrowing the field of vision through the use of coloured sections.
- Indicator for vehicles that prevents double images (Japanese patent document no. 5 193 397).
- Vision system for the outside mirror of a vehicle (Japanese patent document no. 1 031 5763) that avoids glare due to vehicles in the rear.
- Image emission system with a reduced emission angle (French patent document no. 2 811 089) that avoids reflections for use in aeroplane cockpits.
- Phosphate glass containing copper for use in aeroplane cockpits (U.S. Pat. No. 6,252,702). This glass attenuates infrared radiation avoiding the risk of temporary blindness in the pilot without limiting visibility.
- Optical media and procedure for improving or modifying colour vision and method for preparing them (U.S. Pat. No. 5,774,202) using a colour filter with a specific transmission range. For use on any type of surface including glass.
- Visual discerning filter (Japanese patent document no. 6 108 7106) to avoid the change in luminosity that follows a change in tone and reduce the eye effort by providing the maximum absorption possible.
- Ultraviolet filter and glass comprised of this filter (Japanese patent document no. 1 002 0347), whose transmittance of this type of radiation is variable and adjustable.
- Electromagnetic filter (Japanese patent document no. 2000 349542 and 2000 349541) that protects against certain magnetic frequencies received, for example, through windows.
- Absorption filter for color exposure systems (U.S. Pat. No. 5,121,030) that, through the application of dyes, improves visibility in conditions of high luminous intensity.
- Colour highlighting filter and method of use to improve human vision (U.S. Pat. No. 6,158,865). Various embodiments of the invention comprises a filter that improves vision in all light environments including extreme ambient light and low illumination levels, and incorporates an adapting ring for the filter.
- Special optical filters for certain activities and optical accessories that use these filters (U.S. Pat. No. 6,893,127) to improve the visualization of objects, for example in sports activities.

These devices differ from various embodiments of the present invention mainly in their purpose and utility since none has been designed as a prevention/protection device for eyes against the harmful effects of short wavelengths of light. Moreover, most of these patents do not refer to the application of a filter to the transparent surface/s of vehicles rather they are designed as other formats (e.g. specific light systems or solutions).

### Brief description of the invention

The objective of the invention is the prevention and protection of eyes against the absorption of blue and ultra violet light by applying a filter to the transparent or translucent surface(s) of a motor vehicle. As mentioned, it is particularly useful in the case of pseudoaphakic subjects, to functionally compensate for the removal of protective pigments (along with the natural lens during surgery) and in the case of neurodegeneration processes to potentiate the prophylactic effect (processes that often coexist in elderly persons), but is equally important for the protection of healthy eyes in subjects of any age.

The present invention is directed to a component for protecting eyes against short wavelengths of light. In various embodiments of the invention, the component comprises a filter adapted for placement on or inclusion in a transparent or translucent element of a vehicle, the filter containing a yellow pigment that absorbs wavelengths in the range 500 to 380 nm. The filter may be included within the transparent or translucent element of the vehicle or placed on a surface of the transparent or translucent surface of the vehicle. The vehicle element may be the glass used in motor cars, buses, trains, planes, or other transport vehicles. In a preferred embodiment, the filter is transparent so as not to interfere with the viewing through the vehicle element. The component combines:
The transparent or translucent surfaces of any vehicle (cars, buses, trains, planes and so on).
- A mount or device or support for applying the filter in the translucent surface.
- The application of a filter with a commercial yellow pigmentation, compatible with the surface material, said filter being capable to absorb short wavelengths from 380nm to 500nm, in the whole light transmission area of the surface.

### Preferred embodiment of the invention

There are several embodiments of the present invention depending on the specific material of the surface wherein the filter is to be applied. Additionally, the preferred embodiment is illustrated with the following example that is not limitative, due to the existence of different ways and alternative combinations for manufacturing the component.

Example of manufacturing the invention:
- It is prepared a yellow filter among of those available in the market, for example, in the form of film or dye, compatible with the surface wherein is to be applied.
- It is prepared a support material or device among of those available in the market for applying the filter in the transparent or translucent surfaces of the vehicle, according with the manufacturer instructions.
- It is mounted or applied the yellow filter in the surface, occupying the whole light transmission area.

In summary, by applying a yellow filter to the transparent or translucent surface/s of the vehicle in which the subject may be found, any person will be able to protect his/her healthy eyes from short-wavelengths of light. In patients who have undergone cataract surgery with the implant of an intraocular lens, various embodiments of the invention will compensate for the lack of protection of the operated eye and in eyes suffering neurodegenerative processes it will improve and increase their natural protection. This simple precaution will avoid the problems related to the technical options available on the market (filters with no application device and intraocular lenses).

## Claims

1. A component for protecting eyes against short wavelengths of light, **characterized in that** it is the result of the combination of the placement of a filter containing a yellow pigment that absorbs wavelengths in the range 500 to 380 nm in any transparent or translucent surface of a vehicle.

2. The component according to claim 1, **characterized in that** it comprises a filter with yellow pigmentation suitable to be used in the transparent or translucent surface of the vehicle.

3. The component as claimed in claim 1 and 2, wherein the filter is placed on a surface of the transparent or translucent surface of the vehicle.

4. The component as claimed in claim 3, wherein the element is glass used in motor cars, buses, trains, planes, or other transport vehicles.

5. The component as claimed in claim 1, 2, 3 y 4, **characterized in that** is transparent and filtering.

6. A therapeutic or prophylactic component for protecting eyes, **characterized in that** it combines a component comprising a filter containing a yellow pigment that absorbs wavelengths in the range 500 to 380 nm applied to any transparent or translucent surfaces of a vehicle.

7. The component according to claim 6, wherein the filter contains a yellow pigment usable in the transparent or translucent surface.

8. The component according to claim 6 and 7, **characterized in that** it comprises one or more transparent or translucent surfaces of a vehicle.

9. The component according to claim 8, wherein the surface is glass used in motor cars, buses, trains, planes, or any other transport vehicles.

10. The component according to claim 6, 7, 8 and 9, wherein the component is transparent and filtering.

11. A therapeutic and prophylactic component for eyes with a retinal neurodegenerative processes **characterized in that** it combines a filter material containing a yellow pigment that absorbs wavelengths in the range 500 to 380 nm applied to any transparent or translucent surfaces of a vehicle.

12. The component according to claim 11, **characterized in that** it comprises a filter with yellow pigmentation suitable to be used in the transparent or translucent surface of the vehicle.

13. The component according to claim 11 and 12, **characterized in that** it comprises one or more transparent or translucent surfaces of the vehicle.

14. The component according to claim 13, wherein the one or more transparent or translucent surfaces of the vehicle is glass used in motor cars, buses, trains, planes or any other transport vehicles.

15. The method according to claim 11, **characterized in that** it is filtering and transparent.
